Europäisches Patentamt

European Patent Office

Office européen des brevets

(11) Veröffentlichungsnummer: **0 519 169 A1**

## EUROPÄISCHE PATENTANMELDUNG

(21) Anmeldenummer: **92105520.8**

(22) Anmeldetag: **31.03.92**

(51) Int. Cl.5: **A61N 2/08**, A44C 9/00

(30) Priorität: **18.05.91 DE 4116357**

(43) Veröffentlichungstag der Anmeldung:
**23.12.92 Patentblatt 92/52**

(84) Benannte Vertragsstaaten:
**AT CH DE FR GB IT LI NL PT SE**

(71) Anmelder: **Rheinmagnet Horst Baermann GmbH**
**Ohlenhohnstrasse 23**
**W-5206 Neunkirchen-S. 1(DE)**

(72) Erfinder: **Baermann, Horst**
**Branderhof 9**
**W-5060 Bergisch Gladbach 1(DE)**

(74) Vertreter: **Stachow, Ernst-Walther et al**
**Lippert, Stachow, Schmidt & Partner,**
**Patentanwälte, Frankenforster Strasse**
**135-137, P.O. Box 30 02 08**
**W-5060 Bergisch Gladbach 1(DE)**

(54) **Ringförmiges Element für therapeutische Anwendungen.**

(57) Ein ringförmiges Element für therapeutische Anwendungen mit darin enthaltenem Dauermagnetmaterial ist zum Zwecke der einfachen und kostensparenden Herstellung in beliebiger Größe und zur Herbeiführung einer therapeutisch optimalen Wirkung aus einem einstückigen, ringförmigen Dauermagneten gebildet.

Die Erfindung betrifft eine ringförmiges Element für therapeutische Anwendungen mit darin enthaltenem Dauermagnetmaterial.

Die Verwendung von Dauermagneten im medizinischtherapeutischen Bereich gewinnt eine immer größer werdende Bedeutung. Es ist schon seit längerer Zeit bekannt, daß konstante oder wechselnde Magnetfelder, die auf zu behandelnde Körperstellen einwirken, eine durchblutungsfördernde Wirkung haben und zur Behandlung bestimmter Beschwerden, wie Krämpfe, Prellungen, Zerrungen usw., mit Erfolg angewendet werden können. Neuere wissenschaftliche Untersuchungen haben die Wechselwirkung zwischen einem lebenden Organismus und Magnetfeldern auf eine physikalische Basis gestellt. Dabei hat man insbesondere die Dissoziation von Ionen in elektrolytischen Systemen aufgrund des Hall-Effektes genauer analysiert. Da der Organismus ein sehr komplexes elektrolytisches System darstellt, wird die therapeutische Wirkung von Magnetfeldern unter anderem darauf zurückgeführt, daß ein Ionenstrom in den Blutgefäßen auftritt bzw. das im Krankheitsfall gestörte Potentialgefälle beiderseits der Zellmembranen im gestörten Bereich wieder hergestellt wird.

Die therapeutische Behandlung durch Magnetfelder gewinnt nicht nur durch ihren medizinischen Erfolg sondern auch dadurch immer mehr an Bedeutung, daß auf Bestrahlungen oder die Einnahme von Chemikalien jeder Art verzichtet werden kann. Insbesondere kommt sie daher Allergikern zugute, die heute mehr als 25% der Bevölkerung industrialisierter Länder ausmachen.

Dauermagnete für therapeutische Anwendungen sind bisher in mehreren Ausbildungen, z.B. als flächige Magnete, bekanntgeworden. Unter anderem sind Dauermagnete auch schon in ringförmige Elemente eingearbeitet worden. So ist ein Armreif mit an der radial innenliegenden Seite angeordneten Magneteinlagen bekannt.

Die Herstellung eines solchen Armreifs ist jedoch ein aufwendiges Verfahren und daher mit relativ hohen Kosten verbunden. Außerdem ist die mit einem solchen Armreif erzeugte magnetische Induktion gering, so daß keine therapeutisch optimale Wirkung eintritt. Das als Armreif bekannte ringförmige Element kann auch nicht ohne weiteres beliebig, z.B. auf die Größe eines Fingerringes, verkleiner werden, so daß es in seiner Anwendung beschränkt ist.

Der Erfindung liegt die Aufgabe zugrunde, ein ringförmiges Element der eingangs genannten Art zu schaffen, das unter Vermeidung der obigen Nachteile einfach und in beliebiger Größe hergestellt werden kann und eine therapeutisch optimale Wirkung gewährleistet.

Die Aufgabe wird erfindungsgemäß dadurch gelöst, daß das ringförmige Element aus einem einstückigen, ringförmigen Dauermagneten gebildet ist.

Ein solcher ringförmiger Dauermagnet kann in einem einzigen Arbeitsgang mit relativ einfachen Mitteln und kostensparend hergestellt werden. Durch Verwendung geeigneter, vorzugsweise hochkoerzitiver Dauermagnetmaterialien kann eine therapeutisch optimale Wirkung erzielt werden. Der erfindungsgemäße Dauermagnet ist weiterhin in beliebigen Größen herstellbar, so daß er z.B. als Fingerring oder als Armreif verwendbar ist.

Der erfindungsgemäße, ringförmige Dauermagnet hat den Vorteil, daß bei allen in Frage kommenden Magnetisierungen des Ringes fast jedes in dem von Ring umschlossenen Körperteil verlaufende Blutgefäß die Feldlinien kreuzt, um den durch den Hall-Effekt bewirkten Ionenstrom zu erzeugen. Diese Erkenntnis macht ein ringförmiges Dauermagnetelement erst für die therapeutische Anwendung bedeutsam.

In einer möglichen Ausführung der Erfindung weist der Ringmagnet eine radiale Magnetisierung mit einem an der inneren Umfangsseite angeordneten Pol und einem dazu entgegengesetzten, an der äußeren Umfangsseite angeordneten Pol auf.

Der Ringmagnet kann alternativ oder zusätzlich auch eine axiale Magnetisierung mit einem an der einen Stirnseite angeordneten Pol und einem dazu entgegengesetzten, an der anderen Stirnseite angeordneten Pol aufweisen.

In einer anderen Ausführung weist der Ringmagnet an der inneren Umfangsseite eine zwei- oder mehrpolige, laterale Magnetisierung auf. Die Anzahl der Pole kann dabei gerade oder ungerade sein. Die Pole können gleiche oder ungleiche Breiten und/oder Abstände haben.

Alternativ oder zusätzlich kann der Ringmagnet eine zwei- oder mehrpolige axiale Magnetisierung mit zwei oder mehreren an einer Stirnseite angeordneten Polen aufweisen.

Schließlich kann der Ringmagnet auch diametral magnetisiert sein mit einem in einer Ringhälfte angeordneten Pol und einem dazu entgegengesetzten, in der anderen Ringhälfte angeordneten Pol.

Der erfindungsgemäße Ringmagnet kann geschlossen oder auch mit einem Schlitz versehen sein.

Weiterhin kann der Ringmagnet zu Dekorationszwecken auch eine Beschichtung aus Metall oder Kunststoff aufweisen. Die Beschichtung kann teil- oder vollflächig sein. Als Beschichtungen kommen alle bei Ringen bekannten Beschichtungen, wie z.B. Gold oder Silber, aber auch Lack oder dergleichen, in Frage.

Der erfindungsgemäße Ringmagnet kann auch ein darauf angeordnetes Zierteil aufweisen. Das Zierteil kann durch beliebige, bekannte Verfahren, wie z.B. Aufkleben, Anschrauben oder dergleichen auf dem Ringmagneten befestigt sein.

Das Zierteil kann in einer anderen Ausführung auch aus Dauermagnetmaterial bestehen und einstückig an den Ring angeformt sein.

Als Werkstoffe für den erfindungsgemäßen Ringmagneten kommen insbesondere hochkoerzitive Werkstoffe in Betracht, wie z.B. Barium- oder Strontiumferrit oder Seltenerdmagnetwerkstoffe, wie z.B. NdFeB oder beliebige Kombinationen dieser Werkstoffe.

Diese Werkstoffe können in gesinterter Ausführung vorliegen.

Sie können andererseits auch aus pulverförmigem Material bestehen, das in feiner oder möglichst homogener Verteilung in einem duro- oder thermoplastischen Kunststoffmaterial eingebettet und z.B. durch Spritzen oder Verpressen in die gewünschte Form gebracht ist.

Der erfindungsgemäße Ringmagnet kann auch einzeln oder in Kombination mit mehreren dieser Magnete in Bandagen aller Art oder in Handschuhen und dergleichen durch Einweben, Kleben sowie durch andere bekannte Verfahren starr oder beweglich eingearbeitet sein.

Einige Ausführungsbeispiele der Erfindung werden im folgenden anhand der Zeichnung näher beschrieben. Es zeigen:

Fig. 1    eine stirnseitige Draufsicht auf ein Ausführungsbeispiel des Ringmagneten,

Fig. 2    einen Querschnitt durch ein anderes Auführungsbeispiel des Ringmagneten,

Fig. 3    eine stirnseitige Draufsicht auf ein weiteres Ausführungsbeispiel des Ringmagneten,

Fig. 4    eine stirnseitige Draufsicht auf ein weiteres Ausführungsbeispiel des Ringmagneten,

Fig. 5    einen Querschnitt durch ein weiteres Ausführungsbeispiel des Ringmagneten,

Fig. 6    eine stirnseitige Draufsicht auf ein Ausführungsbeispiel des Ringmagneten und

Fig. 7    eine Seitenansicht eines Ausführungsbeispiels des Ringmagneten.

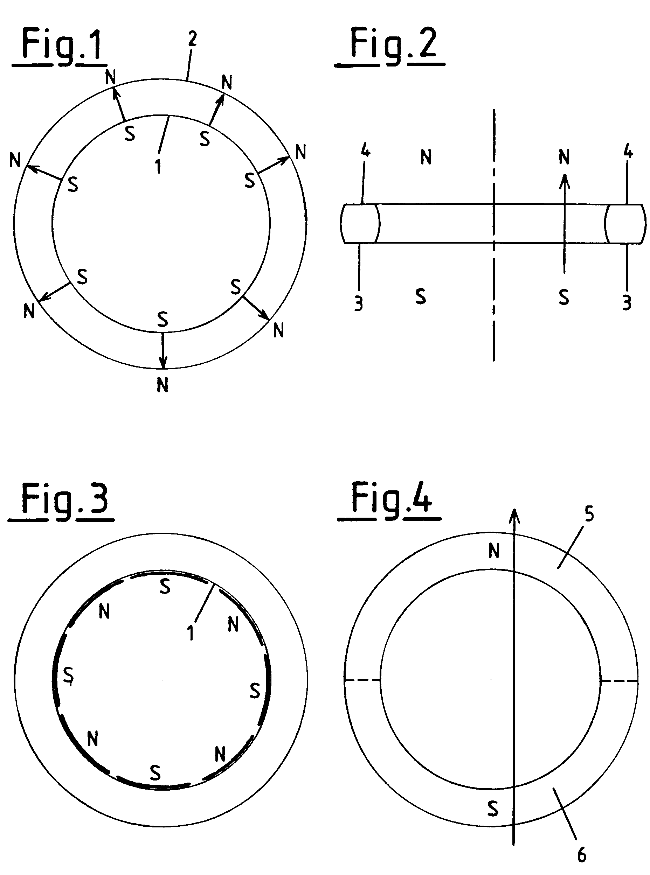

Der in der Zeichnung dargestellte einstückige Ringmagnet kann, wie in den Figuren 1 bis 5 dargestellt ist, auf verschiedene Weise magnetisiert sein.

Bei dem in Fig. 1 dargestellten Ausführungsbeispiel ist eine radiale Magnetisierung vorgesehen, wobei ein Südpol S an der inneren Umfangsseite 1 und ein Nordpol N an der äußeren Umfangsseite 2 des Ringmagneten angeordnet sind.

Fig. 2 zeigt ein Ausführungsbeispiel mit axialer Magnetisierung, wobei ein Südpol S an der einen Stirnseite 3 und ein Nordpol N an der anderen Stirnseite 4 angeordnet sind.

Bei dem in Fig. 3 gezeigten Ausführungsbeispiel ist der Ringmagnet an der inneren Umfangsseite 1 lateral magnetisiert, so daß in dem betrachteten Beispiel vier Nordpole N und vier Südpole S in abwechselnder Folge an der inneren Umfangsseite 1 angeordnet sind.

Fig. 4 zeigt eine diametrale Magnetisierung des Ringmagneten. Dabei besteht die eine Ringhälfte 5 aus einem Nordpol N und die andere Ringhälfte aus einem Südpol S.

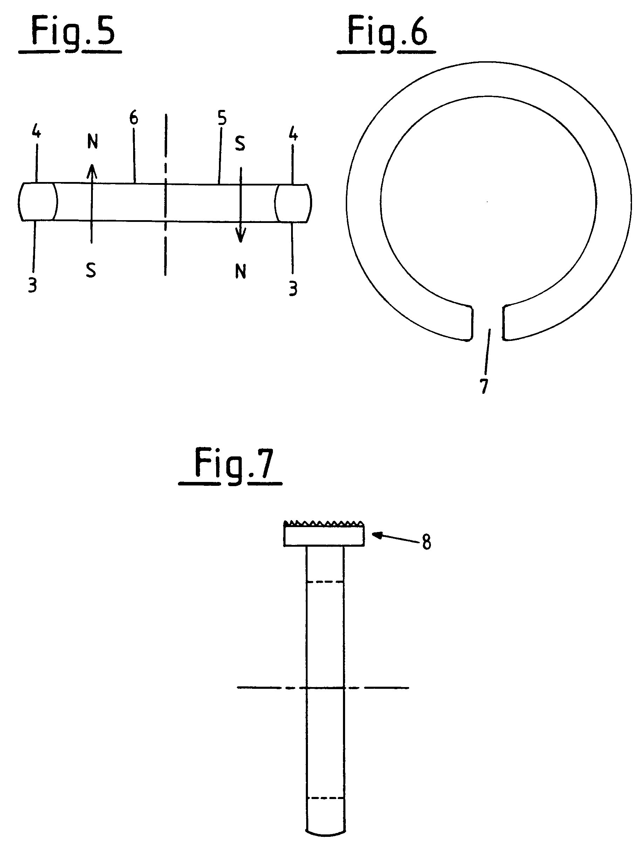

Schließlich ist in Fig. 5 eine zweipolige axiale Magnetisierung des Ringmagneten dargestellt. Die in dieser Figur links gezeigte Ringhälfte 5 weist an der einen Stirnseite 3 einen Nordpol N und an der anderen Stirnseite 4 einen Südpol S auf, während die rechts gezeigte Ringhälfte 6 an der jeweiligen Stirnseite 3 bzw. 4 mit einem Südpol S bzw. einem Nordpol N versehen ist.

Das in Fig. 6 gezeigte Ausführungsbeispiel weist einen Schlitz 7 auf, der ein flexibles Aufbringen des Ringmagneten auf einen Finger, einen Arm oder ein anderes Körperteil ermöglicht.

Fig. 7 zeigt ein weiteres Ausführungsbeispiel, bei dem der Ringmagnet mit einem Zierteil 8 versehen ist. Das Zierteil kann aufgeklebt, angeschraubt oder auf andere Weise auf dem Ringmagneten befestigt sein und aus einem beliebigen Material bestehen. Es kann andererseits auch, z.B. durch Anspritzen, am Ringmagneten einstückig angeformt sein und, wie der Ringmagnet, aus dauermagnetischem Material bestehen.

Die in der Zeichnung dargestellten Ringmagnete können eine teil- oder vollflächige Beschichtung, z.B. aus Metallen, wie Gold oder Silber, oder auch aus Lack oder dergleichen aufweisen.

Die Ringmagnete bestehen aus hochkoerzitiven Werkstoffen, wie z.B. Barium- oder Strontiumferrit oder Seltenerdmagnetwerkstoffen, wie z.B. NdFeB oder Kombinationen dieser Werkstoffe. Sie können in gesinterter Ausführung vorliegen oder aus pulverförmigem Material bestehen, das in feiner oder möglichst homogener Verteilung in einem duro- oder thermoplastischem Material eingebettet ist und z.B. durch Spritzen oder Verpressen in die gewünschte Form gebracht ist.

**Bezugszeichenliste**

| N | Nordpol |
|---|---|
| S | Südpol |
| 1 | innere Umfangsseite |
| 2 | äußere Umfangsseite |
| 3 | Stirnseite |
| 4 | Stirnseite |
| 5 | Ringhälfte |

6     Ringhälfte
7     Schlitz
8     Zierteil

**Patentansprüche**

1. Ringförmiges Element für therapeutische Anwendungen mit darin enthaltenem Dauermagnetmaterial, **dadurch gekennzeichnet, daß** es aus einem einstückigen, ringförmigen Dauermagneten gebildet ist.

2. Ringförmiges Element nach Anspruch 1, **dadurch gekennzeichnet,** daß es eine radiale Magnetisierung mit einem an der inneren Umfangsseite (1) angeordneten Pol (S) und einem dazu entgegengesetzten, an der äußeren Umfangsseite (2) angeordneten Pol (N) aufweist.

3. Ringförmiges Element nach Anspruch 1 oder 2, **dadurch gekennzeichnet,** daß es eine axiale Magnetisierung mit einem an der einen Stirnseite (3) angeordneten Pol (S) und einem dazu entgegensetzten, an der anderen Stirnseite (4) angeordneten Pol (N) aufweist.

4. Ringförmiges Element nach Anspruch 1, **dadurch gekennzeichnet,** daß es an der inneren Umfangsseite (1) eine zwei- oder mehrpolige, laterale Magnetisierung aufweist.

5. Ringförmiges Element nach Anspruch 1 oder 4, **dadurch gekennzeichnet,** daß es eine zwei- oder mehrpolige axiale Magnetisierung mit zwei oder mehreren an einer Stirnseite (3) angeordneten Polen (S,N) und einer entsprechenden Zahl dazu entgegengesetzter, an der anderen Stirnseite (4) angeordneter Pole (N,S) aufweist.

6. Ringförmiges Element nach Anspruch 1, **dadurch gekennzeichnet,** daß es eine diametrale Magnetisierung mit einem in einer Ringhälfte (5) angeordneten Pol (S) und einem dazu entgegengesetzten, in der anderen Ringhälfte (6) angeordneten Pol (N) aufweist.

7. Ringförmiges Element nach einem der Ansprüche 1 bis 6, **dadurch gekennzeichnet,** daß es einen Schlitz (7) aufweist.

8. Ringförmiges Element nach einem der Ansprüche 1 bis 7, **dadurch gekennzeichnet,** daß es eine Beschichtung aus Metall oder Kunststoff aufweist.

9. Ringförmiges Element nach einem der Ansprüche 1 bis 8, **dadurch gekennzeichnet,** daß es einen darauf angeordneten Zierteil (8) aufweist.

10. Ringförmiges Element nach Anspruch 9, **dadurch gekennzeichnet,** daß das Zierteil (8) aus Dauermagnetmaterial besteht und einstückig an den Ring angeformt ist.

11. Ringförmiges Element nach einem der Ansprüche 1 bis 10, **dadurch gekennzeichnet,** daß es aus hochkoerzitiven Werkstoffen, wie Barium- oder Strontiumferriten oder Seltenerdwerkstoffen, wie z.B. NdFeB, oder Kombinationen dieser Werkstoffe besteht.

12. Ringförmiges Element nach Anspruch 11, **dadurch gekennzeichnet,** daß die Werkstoffe in gesinterter Form vorliegen.

13. Ringförmiges Element nach Anspruch 11, **dadurch gekennzeichnet,** daß es aus pulverförmigem Material besteht, das in feiner und möglichst homogener Verteilung in einem duro- oder thermoplastischem Kunststoffmaterial eingebettet und z.B. durch Spritzen oder Verpressen in die gewünschte Form gebracht ist.

14. Ringförmiges Element nach einem der Ansprüche 1 bis 13, **dadurch gekennzeichnet,** daß es einzeln oder in Kombination mit mehreren Elementen in Bandagen aller Art, z.B. in Handschuhen und dergleichen, durch Einweben, Kleben sowie durch andere bekannte Verfahren starr oder beweglich eingearbeitet ist.

## Fig.1

## Fig.2

## Fig.3

## Fig.4

## Fig.5

## Fig.6

## Fig.7

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 400 305 (MALLINCKRODT)<br><br>* Seite 3, Zeile 1 - Seite 4, Zeile 11; Abbildungen *<br>--- | 1,2,4, 11,14 | A61N2/08<br>A44C9/00 |
| X | FR-A-2 611 306 (MARUBENI CORPORATION ET AL.) | 1,3-5,8, 11 | |
| Y | * Seite 8, Zeile 32 - Seite 10, Zeile 31 *<br>* Seite 70, Zeile 16 - Zeile 32; Abbildungen 42-47 *<br>--- | 12 | |
| Y | EP-A-0 036 022 (URAGAMI)<br>* Seite 6, letzter Absatz - Seite 7, Absatz 1 *<br>--- | 12 | |
| X | FR-A-2 626 774 (MERLENT) | 1,9 | |
| Y | * das ganze Dokument *<br>--- | 10 | |
| Y | FR-A-2 117 687 (FIRMA ERICH HERMANN)<br>* Ansprüche 1,3; Abbildungen 1,2 *<br>--- | 10 | |
| X | FR-A-373 320 (ALLPORT ET AL.) | 1,8 | |
| A | * das ganze Dokument *<br><br>----- | 7 | |

| | RECHERCHIERTE SACHGEBIETE (Int. Cl.5) |
|---|---|
| | A61N<br>A44C |

Der vorliegende Recherchenbericht wurde für alle Patentansprüche erstellt

| Recherchenort | Abschlußdatum der Recherche | Prüfer |
|---|---|---|
| DEN HAAG | 30 SEPTEMBER 1992 | Mark Jones |